# EUROPÄISCHE PATENTSCHRIFT

(11) **EP 1 046 639 B1**
(45) Veröffentlichungstag und Bekanntmachung des Hinweises auf die Patenterteilung: **11.12.2002**
(21) Anmeldenummer: 00107126.5
(22) Anmeldetag: 07.04.2000
(51) Int. Cl.: C07D 207/32, B01J 23/44, B01J 23/42, B01J 23/63

(54) **Verfahren zur Herstellung von Pyrrolen**
Process for the preparation of pyrroles
Procédé pour la préparation de pyrroles

(30) Priorität: 23.04.1999 DE 19918568
(43) Veröffentlichungstag der Anmeldung: 25.10.2000
(73) Patentinhaber: BASF AKTIENGESELLSCHAFT, 67056 Ludwigshafen (DE)
(72) Erfinder: Wulff-Döring, Joachim, Dr., 67227 Frankenthal (DE); Simon, Joachim, Dr., 68161 Mannheim (DE); Hesse, Michael, Dr., 67549 Worms (DE); Wahl, Peter, Dr., 68526 Ladenburg (DE)
(74) Vertreter: Isenbruck, Günter, Dr.

(56) Entgegenhaltungen:
- EP-A- 0 067 360
- EP-A- 0 155 649
- EP-A- 0 167 996
- DE-A- 1 795 315
- GB-A- 1 393 086

## Beschreibung

Die vorliegende Erfindung betrifft ein Verfahren zur Herstellung von Pyrrolen aus Pyrrolidin bzw. substituierten Pyrrolidinen durch Dehydrieren an Edelmetallkatalysatoren.

Bisher wurden bei der Herstellung von Pyrrol und substituierten Pyrrolen aus den entsprechenden Pyrrolidinen im wesentlichen Pd-Katalysatoren eingesetzt. Diese zeichnen sich durch eine relativ niedrige Aktivität aus.

In der US-A 3 522 269 wird die Dehydrierung von Pyrrolidin zu Pyrrol an Pd-Katalysatoren bei sehr hohen Temperaturen (bevorzugt 400 - 450°C), beschrieben, wobei die Katalysatoren relativ schnell desaktivieren.

In der GB-A 1 393 086 wird die Dehydrierung von Piperidin zu Pyridin mittels Katalysatoren aus Palladium auf SiO₂ als Träger beschrieben. Die Herstellung der Katalysatoren erfolgt in halogenierten Kohlenwasserstoffen, d.h. unter umweltbelastenden Bedingungen.

In den EP-A 67 360 und 155 649 werden Pd-Katalysatoren zur Dehydrierung von Pyrrolidinen beschrieben, die bei niedrigeren Temperaturen wirksam sind, aber auch verhältnismäßig geringe Aktivität zeigen.

Aufgabe der Erfindung war es, ein Verfahren zur katalytischen Dehydrierung von Pyrrolidin oder substituierten Pyrrolidinen zu den entsprechenden Pyrrolen sowie einen Katalysator für dieses Verfahren vorzuschlagen, die eine hohe Wirksamkeit über längere Zeit gewährleisten und eine umweltfreundliche Arbeitsweise ermöglichen.

Die Erfindung geht aus von einem Verfahren zum Herstellen von Pyrrolen der allgemeinen Formel I 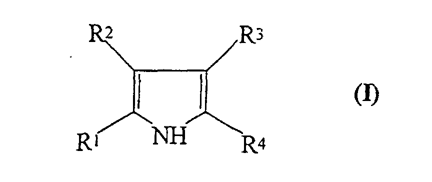 worin R¹, R², R³ und R⁴ gleich oder verschieden sind und Wasserstoffatome, Alkylgruppen mit 1 bis 12 Kohlenstoffatomen oder Cycloalkylgruppen mit 3 bis 12 Kohlenstoffatomen bedeuten, durch Dehydrieren von Pyrrolidinen der allgemeinen Formel II 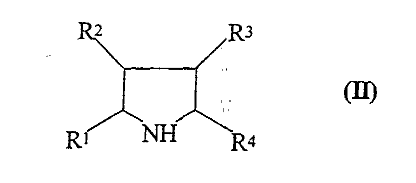 worin R¹, R², R³ und R⁴ die oben genannten Bedeutungen haben, in Gegenwart eines geträgerten Edelmetallkatalysators.

Das erfindungsgemäße Verfahren ist dadurch gekennzeichnet, daß man die Dehydrierung bei Temperaturen im Bereich von 150 bis 300°C und Drücken von 0,01 bis 50 bar durchführt und daß der Edelmetallkatalysator 30 bis 100 Gew.-%
a) Palladium auf einem Oxid eines Elements der 4. Gruppe (IV. Nebengruppe) oder
b) eines Platin/Palladium-Gemischs auf einem Oxid eines Elements der 4. Gruppe und 0 bis 70 Gew.-% Alkali- oder Erdalkalimetalloxid enthält.

Das erfindungsgemäße Verfahren läßt sich wie folgt durchführen:

Die Umsetzung der Pyrrolidine der Formel II zu den Pyrrolen I kann an einem Heterogenkatalysator in der Flüssigphase oder bevorzugt in der Gasphase bei Temperaturen von 150 bis 300°C, bevorzugt 170 bis 270°C, besonders bevorzugt 180 bis 250°C und Drücken von 0,01 bis 50 bar, bevorzugt 0,1 bis 5 bar, besonders bevorzugt von 1 bis 1,5 bar durchgeführt werden.

Stickstoff und Wasserstoff werden - bezogen auf das eingesetzte Pyrrolidin - in der Regel in einem Molverhältnis von 1:1 bis 100:1, bevorzugt 2:1 bis 50:1, besonders bevorzugt 3:1 bis 40:1 eingesetzt. Das Molverhältnis von Stickstoff zu Wasserstoff kann 0,01:1 bis 100:1, bevorzugt 0,1:1 bis 10:1, besonders bevorzugt 0,5:1 bis 5:1 betragen.

Die Reaktion wird bevorzugt in einem Rohrreaktor durchgeführt, da es nur darauf ankommt, eine Rückvermischung vom Ausgang der Anlage, d.h. dem Ende der letzten Reaktionszone, zum Eingang, d.h. dem Beginn der ersten Reaktionszone, zu vermeiden, was sich in Rohrreaktoren und der sich darin ausbildenden Pfropfenströmung am leichtesten erreichen läßt.

Der Katalysator kann im Festbett oder auch im Wirbelbett angeordnet sein. Als maßgeschneiderte Katalysatoren für die zweite Reaktionszone haben sich die erfindungsgemäßen Heterogenkatalysatoren bewährt.

Als Heterogenkatalysatoren eigenen sich solche, die 30 bis 100, bevorzugt 50 bis 100, besonders bevorzugt 70 bis 100 Gew.-%
a) Palladium auf einem Oxid eines Elements der 4. Gruppe (IV. Nebengruppe) des Periodensystems oder
b) eines Platin/Palladium-Gemischs einem Oxid eines Elements der 4. Gruppe und 0 bis 70 Gew.-%, bevorzugt 0 bis 50 Gew.-%, besonders bevorzugt 0 bis 30 Gew.-% eines Alkali- und/oder Erdalkalimetalloxids enthalten.

Als Metalle der 4. Gruppe eignen sich unter anderem Titan, Zirkonium und Hafnium, bevorzugt Titan und Zirkonium, besonders bevorzugt Zirkonium.

Als Alkali- oder Erdalkalimetalle eignen sich zum Beispiel Lithium, Natrium, Kalium, Caesium, Beryllium, Magnesium, Calcium, Strontium und Barium, bevorzugt Natrium, Kalium, Magnesium, Calcium und Barium.

Die aktiven Bestandteile des Katalysators (Edelmetalle) befinden sich, wenn es sich um reines Palladium handelt, vorzugsweise auf Oxiden von Elementen der 4. Gruppe, und, wenn es sich um Platin/Palladium-Gemische handelt, vorzugsweise auf Oxiden der 4. Gruppe.

Die Katalysatoren können, soweit vorhanden, durch gemeinsames Kneten der Zusätze (d.h. der Alkali- oder Erdalkalimetalloxide) mit dem Trägermaterial, thermische Nachbehandlung (Tempern) bei 400 bis 900°C und Tränken mit einer ein Salz des Edelmetalls enthaltenden Lösung oder durch Tränken des Trägers mit einer Lösung der Zusätze und des Edelmetalls, z.B. in Form von Lösungen ihrer Nitrate, Chloride, Formiate, Oxalate oder Ammoniakate und darauf folgendes Tempern bei 400 bis 900°C hergestellt werden. Wenn eine Spinellbildung bewirkt werden soll, muß nach dem Kneten oder dem Tränken des Aluminiumoxids mit dem Oxid bzw. der Lösung der Zusatzkomponente eine Temperatur von 900 bis 1.300°C erreicht werden (siehe Ullmanns Encyklopädie der technischen Chemie, 3. Auflage (1955) Band 6, Seiten 242 bis 244).

Der Edelmetallgehalt des Katalysators, bezogen auf das Trägermaterial, liegt in der Regel bei 0,0001 bis 25 Gew.-%, bevorzugt 0,001 bis 20 Gew.-%, besonders bevorzugt 0,05 bis 15 Gew.-%. Man kann die Katalysatoren beispielsweise in Form von Formkörpern, z.B. Strängen, Tabletten oder Ringen, oder als Pulver je nach dem vorgesehenen Verwendungszweck einsetzen.

Das erfindungsgemäße Verfahren hat gegenüber den bekannten Verfahren den Vorteil, daß durch den Einsatz von aktiveren und langsamer desaktivierenden Katalysatoren bei der Dehydrierung im technischen Betrieb deutlich weniger Unterbrechungen oder Stillstandszeiten zum Katalysatorwechsel erforderlich werden und dadurch die Kapazität erhöht wird. Darüber hinaus haben die erfindungsgemäß eingesetzten Katalysatoren eine höhere Anfangsselektivität für die Synthese zum aromatischen Heterocyclus, so daß die Selektivität über die gesamte Laufzeit mit einer Katalysatorcharge deutlich besser ist.

Die Bildung von häufig auftretenden typischen Nebenprodukten, wie Pyrrolinen u.a. kann bei dem erfindungsgemäßen Verfahren weitgehend unterdrückt werden, so daß sich keine oder nur geringe Mengen dieser Nebenprodukte bilden.

Die Substituenten R¹, R², R³, R⁴ und R⁵ in den Formeln I und II haben folgende Bedeutungen:
- Wasserstoff,
- C₁- bis C₁₂-Alkyl, bevorzugt C₁₋ bis C₈-Alkyl, besonders bevorzugt C₁₋ bis C₄-Alkyl, z.B. Methyl, Ethyl, Isopropyl, n-Butyl, sec.-Butyl und tert.-Butyl,
- C₃- bis C₁₂-Cycloalkyl, bevorzugt C₅- bis C₈-Cycloalkyl, besonders bevorzugt C₅- oder C₆-Cycloalkyl, wobei diese Reste gegebenenfalls durch 1 bis 3 Substituenten, wie Halogenatome oder C₁-C₄-Alkylgruppen, substituiert sein können.

Die nach dem Verfahren der Erfindung hergestellten Verbindungen können z.B. zur Herstellung von pharmazeutischen Wirkstoffen eingesetzt werden und stellen wertvolle Zwischenprodukte für deren Synthese dar.

### Beispiel 1 (Vergleichsbeispiel gemäß EP-A 67 360):

8 kg Aluminiumoxid wurden mit 2 kg Magnesiumoxid (jeweils berechnet auf reines Al₂O₃ und MgO) unter Zugabe von etwa 10 Litern Wasser geknetet und anschließend im Extruder zu 4 mm dicken Strängen verformt. Die erhaltenen Stränge wurden bei 120°C 6 Stunden getrocknet und danach bei 450°C 2 Stunden getempert.

Die Stränge wurden in einer Imprägniertrommel mit einer Pd-nitratlösung von 5 Gew.-% getränkt, indem die Lösung heiß auf die Stränge gesprüht wurde. Die Stränge wurden dann bei 120°C 4 Stunden getrocknet und danach bei 520°C 2 Stunden calziniert.

Der in dieser Weise entsprechend EP-A 67 360 hergestellte Katalysator A enthielt 1 Gew.-% Pd auf einem Träger, der aus 19,4 Gew.-% Magnesiumoxid und 80,6 Gew.-% Aluminiumoxid bestand.

In ein elektrisch beheizbares 2,5 l fassendes Reaktorrohr von 1 m Länge wurden 750 ml Katalysator eingefüllt. Der Reaktor wurde dann im Stickstoffstrom (100 1 je Stunde) auf 180°C (5°C je Minute) aufgeheizt und bei dieser Temperatur gehalten. Während der nächsten 4 Stunden wurden 30 l Wasserstoff je Stunde zudosiert. Anschließend wurde der Stickstoff über 4 Stunden allmählich durch Wasserstoff ersetzt, bis nur noch Wasserstoff durch den Reaktor geleitet wurde. Dann wurde die Temperatur zunächst auf 200°C gebracht, 2 Stunden auf dieser Höhe gehalten, anschließend auf 220°C erhöht und erneut 2 Stunden dort gehalten. Der aktivierte Katalysator wurde direkt für den Versuch eingesetzt und bis Versuchsbeginn unter Stickstoff gehalten.

Die Reaktion wurde unter Normaldruck im Gasstrom (100 Normliter je Stunde Stickstoff, 30 Normliter je Stunde Wasserstoff) durchgeführt. Zum Gasstrom wurden 0,06 kg Zulauf (Pyrrolidindampf) je Liter Katalysator je Stunde über den Verdampfer eindosiert. Nach dem Reaktor wurden die flüssigen Reaktionsprodukte über einen zweistufigen Intensivkühler mit nachgeschalteter Kühlfalle auskondensiert und gaschromatographisch analysiert. Nach Ende des Versuchstages wurde noch 1 Stunde mit Ammoniak/Wasserstoff gespült und schließlich unter Stickstoff abgekühlt.

Die Versuchsergebnisse sind in der nachstehenden Tabelle 1 zusammengefaßt.

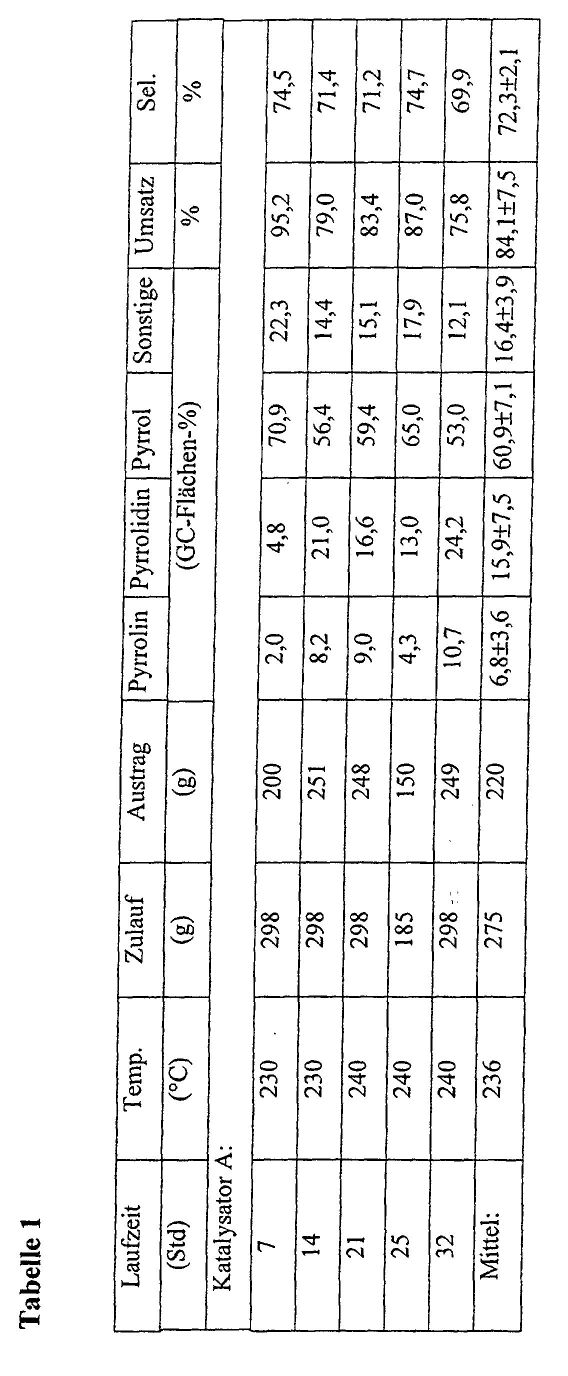

Sel. bedeutet Selektivität, d.h. Ausbeute an Pyrrol, bezogen auf umgesetztes Pyrrolidin. Die Ausbeuten wurden durch Gaschromatographie bestimmt und in Flächenprozent angegeben. Im Verlauf des Versuchs mußte die Temperatur zur Erhaltung des Aktivitätsniveaus erhöht werden, d.h. die Wirksamkeit bei gleichbleibender Temperatur ließ nach.

### Beispiel 2

Es wurde wie in Beispiel 1 verfahren, aber der Katalysator A durch den Katalysator B ersetzt. Katalysator B wurde entsprechend Katalysator A hergestellt, jedoch wurden ZrO₂-Stränge als Träger und anstelle der Pd-nitrat-Lösung eine Lösung verwendet, die gleiche Mengen Pd-nitrat und Pt-nitrat enthielt. Der so hergestellte Katalysator enthielt 0,5 Gew.-% Pt und 0,5 Gew.-% Pd.

Die Versuchsergebnisse sind in der nachstehenden Tabelle 2 zusammengefaßt.

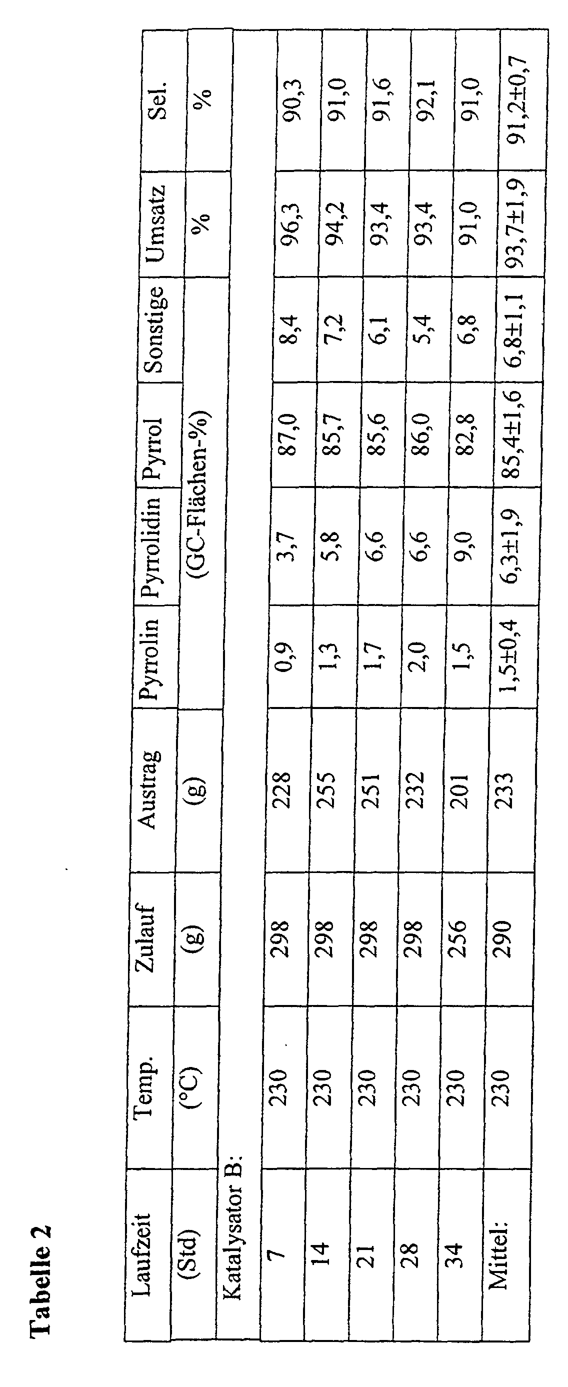

Man erkennt, daß der Katalysator B bei gleichen Umsätzen deutlich höhere Pyrrolmengen liefert. Auch ist er wesentlich aktiver, was an der niedrigeren Reaktionstemperatur zu erkennen ist; und die Standzeit ist höher.

## Patentansprüche

1. Verfahren zum Herstellen von Pyrrolen der allgemeinen Formel I 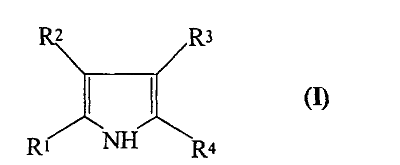 worin R¹, R², R³ und R⁴ gleich oder verschieden sind und Wasserstoffatome, Alkylgruppen mit 1 bis 12 Kohlenstoffatomen oder Cycloalkylgruppen mit 3 bis 12 Kohlenstoffatomen bedeuten, durch Dehydrieren von Pyrrolidinen der allgemeinen Formel II 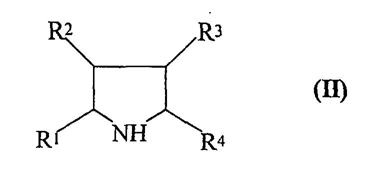 worin R', R², R³ und R⁴ die oben genannten Bedeutungen haben, in Gegenwart eines geträgerten Edelmetallkatalysators, **dadurch gekennzeichnet, daß** man die Dehydrierung bei Temperaturen im Bereich von 150 bis 300°C und Drücken von 0,01 bis 50 bar durchführt und daß der Edelmetallkatalysator 30 bis 100 Gew.-%
a) Palladium auf einem Oxid eines Elements der 4. Gruppe (IV. Nebengruppe) des Periodensystems oder
b) eines Platin/Palladium-Gemischs auf einem Oxid eines Elements der 4. Gruppe.
und 0 bis 70 Gew.-% Alkali- oder Erdalkalimetalloxid enthält.

2. Verfahren nach Anspruch 1, **dadurch gekennzeichnet, daß** man die Umsetzung in der Flüssigphase oder in der Gasphase durchführt.

3. Verfahren nach Anspruch 1, **dadurch gekennzeichnet, daß** man die Umsetzung in Gegenwart von Wasserstoff in einer Menge von 1 bis 100 mol je mol Pyrrolidin durchführt.

4. Verfahren nach einem der Ansprüche 1 bis 3, **dadurch gekennzeichnet, daß** man als Oxid eines Elements der 4. Gruppe ein Oxid des Titans oder Zirkoniums einsetzt.

5. Verfahren nach einem der Ansprüche 1 bis 4, **dadurch gekennzeichnet, daß** man als Alkali- oder Erdalkalimetalloxid ein Oxid des Natriums, Kaliums, Magnesiums, Calciums oder Bariums einsetzt.

6. Verfahren nach einem der Ansprüche 1 bis 5, **dadurch gekennzeichnet, daß** der Edelmetallgehalt des Katalysators, bezogen auf das Trägermaterial, im Bereich von 0,0001 bis 25 Gew.-% liegt.

7. Verfahren nach einem der Ansprüche 1 bis 6, **dadurch gekennzeichnet, daß** einer der Reste R¹, R², R³ und R⁴ ein Wasserstoffatom oder eine C₁-C₄-Alkylgruppe ist und die übrigen Wasserstoffatome sind.

## Claims

1. A process for preparing pyrroles of the formula I 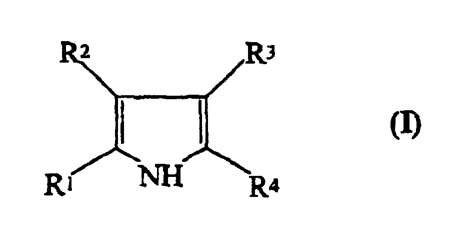 where R¹, R², R³ and R⁴ are identical or different and are hydrogen atoms, alkyl groups having from 1 to 12 carbon atoms or cycloalkyl groups having from 3 to 12 carbon atoms, by dehydrogenation of pyrrolidines of the formula II 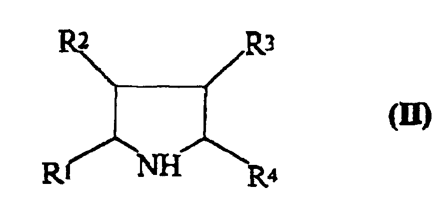 where R¹, R², R³ and R⁴ are as defined above, in the presence of a supported noble metal catalyst, wherein the dehydrogenation is carried out at from 150 to 300°C and pressures of from 0.01 to 50 bar and the noble metal catalyst comprises from 30 to 100% by weight of
a) palladium on an oxide of an element of the 4^{th} group (transition group IV) of the Periodic Table, or
b) a platinum/palladium mixture on an oxide of an element of the 4^{th} group
and from 0 to 70% by weight of alkali metal oxide or alkaline earth metal oxide.

2. A process as claimed in claim 1, wherein the reaction is carried out in the liquid phase or in the gas phase.

3. A process as claimed in claim 1, wherein the reaction is carried out in the presence of hydrogen in an amount of from 1 to 100 mol per mol of pyrrolidine.

4. A process as claimed in any of claims 1 to 3, wherein an oxide of titanium or zirconium is used as oxide of an element of the 4^{th} group.

5. A process as claimed in any of claims 1 to 4, wherein an oxide of sodium, potassium, magnesium, calcium or barium is used as alkali metal oxide or alkaline earth metal oxide.

6. A process as claimed in any of claims 1 to 5, wherein the noble metal content of the catalyst, based on the support material, is in the range from 0.0001 to 25% by weight.

7. A process as claimed in any of claims 1 to 6, wherein one of the radicals R¹, R², R³ and R⁴ is a hydrogen atom or a C₁-C₄-alkyl group and the others are hydrogen atoms.

## Revendications

1. Procédé pour la préparation de pyrroles de la formule générale I 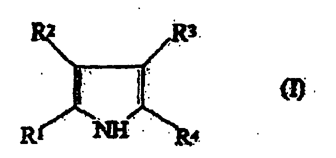 dans laquelle R¹, R², R³ et R⁴ sont identiques ou différents et représentent des atomes d'hydrogène, des radicaux alkyle comportant de 1 à 12 atomes de carbone ou des radicaux cycloalkyle comportant de 3 à 12 atomes de carbone, par déshydrogénation de pyrrolidines de la formule générale II 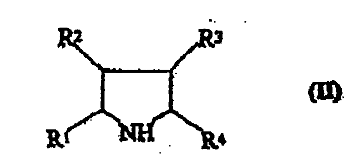 dans laquelle R¹, R², R³ et R⁴ ont les significations ci-dessus, en présence d'un catalyseur supporté à base de métal noble, **caractérisé en ce que** l'on entreprend la déshydrogénation à des températures de l'ordre de 150 à 300°C et des pressions de 0,01 à 50 bar et que le catalyseur de métal noble contient de 30 à 100% en poids
a) de palladium sur un oxyde d'un élément du 4^{ème} groupe (IVème sous-groupe) du système périodique ou
b) un mélange de platine / palladium sur un oxyde d'un élément du 4^{ème} groupe,
et de 0 à 70% en poids d'un oxyde de métal alcalin ou alcalino-terreux.

2. Procédé selon la revendication 1, **caractérisé en ce que** l'on entreprend la réaction dans la phase liquide ou dans la phase gazeuse.

3. Procédé selon la revendication 1, **caractérisé en ce que** l'on entreprend la réaction en présence d'hydrogène en une quantité de 1 à 100 moles par mole de pyrrolidine.

4. Procédé selon l'une des revendications 1 à 3, **caractérisé en ce que** l'on met en oeuvre en tant qu'oxyde d'un élément du 4^{ème} groupe un oxyde de titane ou de zirconium.

5. Procédé selon l'une des revendications 1 à 4, **caractérisé en ce que** l'on met en oeuvre en tant qu'oxyde de métal alcalin ou alcalino-terreux un oxyde de sodium, de potassium, de magnésium, de calcium ou de baryum.

6. Procédé selon l'une des revendications 1 à 5, **caractérisé en ce que** la teneur en métal noble du catalyseur, par rapport au matériau de support, est de l'ordre de 0,0001 à 25% en poids.

7. Procédé selon l'une des revendications 1 à 6, **caractérisé en ce que** l'un des restes R¹, R², R³ et R⁴ est un atome d'hydrogène ou un radical alkyle en C₁ à C₄ et les autres sont des atomes d'hydrogène.
